# EUROPEAN PATENT APPLICATION

(11) **EP 3 338 841 A1**
(43) Date of publication of application: **27.06.2018**
(21) Application number: 16206713.6
(22) Date of filing: 23.12.2016
(51) Int. Cl.: A61M 11/00, A61M 15/00, B01L 3/02, B05B 11/00, A61J 1/06, A23L 33/00, A23L 33/175

(54) **LIQUID HUMAN MILK SUPPLEMENT**

(71) Applicant: Haschke, Ferdinand, 1814 La Tour-de-Peilz (CH)
(72) Inventor: Haschke, Ferdinand, 1814 La-Tour-de-Peilz (CH); Van Goudouver, Hans J., 1081 Amsterdam (NL)
(74) Representative: Lang, Johannes

(57) **Abstract**

A liquid human milk supplement comprising at least the amino acids leucine, isoleucine, valine, lysine, methionine, threonine, phenylalanine, histidine, and tryptophan, with the total amount of amino acids comprising at least 25% of the caloric content of the supplement. The supplement is particularly suitable for feeding infants and low-birth-weight infants. A single-dose and a multi-dose dispenser comprise the supplement in suitable and adjustable volumes. The dispensers can be used for administration of the liquid human milk supplement to an infant during feeding.

## Description

The present invention relates to a liquid human milk supplement, comprising a mixture of amino acids. Further, the present invention relates to the use of the liquid human milk supplement for feeding infants, in particular low-birth-weight infants. The present invention also provides a single-dose and a multi-dose dispenser comprising the liquid human milk supplement.

### 1. Field of the invention

The present invention relates to a liquid human milk supplement (fortifier), in particular to an amino acid-based liquid human milk supplement for low-birth-weight (LBW) infants. The supplement comprises essential amino-acids, preferably further semi-essential amino acids and optionally non-essential amino acids. The supplement may further comprise lipids, vitamins, minerals and trace elements. The supplement may be delivered by multi-dose or single-dose dispensers. Delivery of the liquid human milk supplement to LBW infants >1500-1800g does not interfere with breastfeeding, because the single dose dispenser allows to give the fortifier directly into the mouth of the LBW infant before, during, and after breastfeeding.

### 2. Prior art

LBW infants are categorized as those infants weighing less than 2500 grams at birth and include two weight subcategories [1] (numbers in squared brackets refer to the reference list at the end of the description): extremely low birth weight infants (ELBW; birthweight <1000g) and very low birth weight infants (VLBW; birthweight <1500g). Many LBW infants, in particular in developing countries have intrauterine growth restriction (SGA, IUGR), usually due to poor maternal nutrition and/or maternal health, to problems with the placenta, or to birth defects. Many LBW infants with IUGR are preterm (i.e. born <37 weeks gestational age) and thus are both physically small and physiologically immature. The percentage of newborns who are LBW is around 8-12% in most countries. Globally, about 18-20 millions of LBW infants are born each year. In South Asia, the percentage is as high as 25% (i.e. 6-7 millions of LBW each year).

For LBW- and term infants, human milk is generally recognized as the ideal feeding due to its overall nutritional composition. Human milk provides infants with unique nutritional-, immune protection-, and developmental benefits and is therefore superior to commercial infant formulas. For feeding of LBW infants, milk from their mothers and/or donor human milk from milk banks should be the first feeding choice [3, 4, 5]. Once full enteral feeding is tolerated and established, LBW infants consume approximately 150 ml (max. 180 ml) human milk per kilogram of body weight [3, 4]. They have specific nutritional requirements that are related to their rapid growth after birth [2], their underdeveloped digestive system and developmental immaturity [e.g. brain; 3, 4, 5]. Therefore, human milk does not meet nutritional needs of LBW infants, in particular of VLBW and ELBW infants. International Nutrition Committees agree that human milk is too low in its protein-, caloric- and mineral content and needs to be fortified when fed to LBW infants [3, 4, 5]. Regulatory aspects have been published in the Official Journal of the European Union; Delegated Regulation; L 25/30 - L 25/43; 2.2.2016. Approximately annually 12-15 millions of LBW infants who receive human milk can benefit from nutritional supplements until they reach a body weight of 2500-3000grams. LBW infants who receive human milk and supplements (fortifiers) show faster length and weight gain which is related to more favorable neurodevelopmental outcome later in life.

Protein requirements of LBW infants, in particular for gain of lean body mass, are substantially higher than in term infants [6]. Human milk supplements with intact bovine milk protein (casein-, whey based), acidified protein, or hydrolyzed protein have been developed. However, there is still a demand for a tailor made human milk supplement for LBW infants.

### 3. Brief description of the invention

This objective is solved by the liquid human milk supplement of the present invention. The supplement includes a targeted blend of essential and preferably semi-essential amino acids (the basic building blocks needed for protein synthesis, -gain, and -turnover). According to inventor's knowledge such supplements for LBW infants are not known in the literature and are not on the market.

Accordingly, the present invention relates to a liquid human milk supplement comprising at least the amino acids leucine, isoleucine, valine, lysine, methionine, threonine, phenylalanine, histidine, and tryptophan,
wherein the total amount of amino acids comprises at least 25 % of the caloric content of the liquid human milk supplement.

Preferably, the total amount of amino acids comprises at least 40% of the caloric content of the liquid human milk supplement, in particular at least 50 % of the caloric content of the liquid human milk supplement.

In another embodiment, the total amount of amino acids may comprise at least 60 % of the caloric content of the liquid human milk supplement. In another embodiment, the total amount of amino acids may comprise at least 70 % of the caloric content of the liquid human milk supplement. In yet another embodiment, the total amount of amino acids may comprise at least 80 % of the caloric content of the liquid human milk supplement. In another embodiment, the total amount of amino acids may comprise at least 90 % of the caloric content of the liquid human milk supplement. In another embodiment, the total amount of amino acids may comprise at least 99 % of the caloric content of the liquid human milk supplement. In yet another embodiment, the total amount of amino acids may comprise 100 % of the caloric content of the liquid human milk supplement.

In one embodiment, the supplement may consist of amino acids with micronutrients in aqueous solution. In another embodiment, the supplement may consist of amino acids without micronutrients in aqueous solution.

In another embodiment, the liquid human milk supplement may have a caloric density in a range of 0.15 kcal/ml to 4 kcal/ml. Preferably, the liquid human milk supplement may have a caloric density in a range of 0.2 kcal/ml to 3 kcal/ml, more preferably in a range of 0.3 to 2.5 kcal/ml.

The liquid human milk supplement may further comprise at least one semi-essential amino acid, in particular at least one amino acid selected from tyrosine, cysteine, glycine, glutamic acid and arginine.

Preferably, at least two, three, or four of these amino acids are comprised. In a particularly preferred embodiment, the supplement comprises tyrosine, cysteine, glycine, glutamic acid and arginine.

The liquid human milk supplement may further comprise at least one non-essential amino acid, in particular at least one amino acid selected from alanine, aspartic acid, proline and serine.

Preferably, at least two, or three of these amino acids are comprised. In a particularly preferred embodiment, the supplement comprises alanine, aspartic acid, proline and serine.

In a preferred embodiment, the liquid human milk supplement comprises the essential amino acids leucine, isoleucine, valine, lysine, methionine, threonine, phenylalanine, histidine, and tryptophan, and further the amino acids tyrosine, cysteine, glycine, glutamic acid, arginine, alanine, aspartic acid, proline and serine.

The amino acids may be present in the following amounts per 30 ml liquid human milk supplement:
leucine (LEU) in a range of 50 to 503 mg, preferably 100 to 400 mg, more preferably 150 to 300 mg,
isoleucine (ILE) in a range of 25 to 270 mg, preferably 55 to 210 mg, more preferably 80 to 160 mg,
valine (VAL) in a range of 35 to 380 mg, preferably 75 to 305 mg, more preferably 115 to 230 mg,
lysine (LYS) in a range of 50 to 515 mg, preferably 100 to 410mg, more preferably 150 to 307 mg,
threonine (THR) in a range of 25 to 265 mg, preferably 52 to 210 mg, more preferably 78 to 157 mg,
phenylalanine (PHE) in a range of 29 to 295 mg, preferably 58 to 235 mg, more preferably 87 to 175 mg,
methionine (MET) in a range of 19 to 200 mg, preferably 40 to 156 mg, more preferably 58 to 117 mg,
histidine (HIS) in a range of 18 to 190 mg, preferably 37 to 150 mg, more preferably 56 to 112 mg, and
tryptophan (TRP) in a range of 13 to 130 mg, preferably 26 to 104 mg, more preferably 39 to 78 mg.

The further amino acids, if present, may be present in the following amounts per 30 ml liquid human milk supplement:
tyrosine (TYR) in a range of 11 to 115 mg, preferably 23 to 92 mg, more preferably 34 to 69 mg,
cysteine (CYS) in a range of 8 to 88 mg, preferably 17 to 70 mg, more preferably 26 to 52 mg,
glycine (GLY) in a range of 50 to 520 mg, preferably 100 to 410 mg, more preferably 150 to 310 mg,
glutamic acid (GLU) in a range of 50 to 528 mg, preferably 105 to 420 mg, more preferably 160 to 315 mg,
arginine (ARG) in a range of 35 to 365 mg, preferably 72 to 290 mg, more preferably 110 to 215 mg,
alanine (ALA) in a range of 25 to 255 mg, preferably 50 to 204 mg, more preferably 75 to 155 mg,
aspartic acid (ASP) in a range of 15 to 163 mg, preferably 30 to 130 mg, more preferably 50 to 100 mg,
proline (PRO) in a range of 29 to 293 mg preferably 58 to 235 mg, more preferably 87 to 175 mg, and
serine (SER) in a range of 7 to 78 mg, preferably 15 to 62, more preferably 23 to 46 mg.

In another embodiment, intact proteins may be absent from the liquid human milk supplement. In another embodiment, hydrolyzed proteins may be absent from the liquid human milk supplement. In another embodiment, partially hydrolyzed proteins may be absent from the liquid human milk supplement. In another embodiment, peptides may be absent from the liquid human milk supplement.

In another embodiment, the liquid human milk supplement may further comprise carbohydrates and fatty acids, wherein the caloric content of the carbohydrates and fatty acids is not greater than 75 % of the total caloric content, preferably not greater than 50 % of the total caloric content. In another embodiment, the liquid human milk supplement may further comprise carbohydrates and fatty acids, wherein the caloric content of the carbohydrates and fatty acids not greater than 30 % of the total caloric content, preferably not greater than 20 % of the total caloric content, more preferably not greater than 10 % of the total caloric content. In another embodiment, the liquid human milk supplement may further comprise carbohydrates and fatty acids, wherein the caloric content of the carbohydrates and fatty acids is not greater than 2 % of the total caloric content, preferably wherein the caloric content of the carbohydrates and fatty acids is 0 %.

In another embodiment, lactose may be absent from the liquid human milk supplement. In another embodiment, pectin may be absent from the liquid human milk supplement. In another embodiment, cellulose and/or cellulose derivatives may be absent from the liquid human milk supplement.

In another embodiment, lipids may be absent from the liquid human milk supplement. In another embodiment, micronutrients may be absent from the liquid human milk supplement.

In another embodiment, carbohydrates may be absent from the liquid human milk supplement. In another embodiment, carbohydrates and micronutrients may be absent from the liquid human milk supplement.

In another embodiment, lipids and micronutrients may be absent from the liquid human milk supplement. In another embodiment, carbohydrates and lipids may be absent from the liquid human milk supplement. In another embodiment, carbohydrates, lipids, and micronutrients may be absent from the liquid human milk supplement.

In another embodiment, long chain saturated fatty acids with aliphatic tails of 13 and more carbons may be absent from the liquid human milk supplement. In another embodiment, saturated fatty acids may be absent from the liquid human milk supplement.

In another embodiment, the liquid human milk supplement may comprise at least one selected from phosphoglycerides, medium-chain triglycerides and unsaturated fatty acids as the only fatty acid source. In another embodiment, the liquid human milk supplement may comprise phosphoglycerides, medium-chain triglycerides and unsaturated fatty acids as the only fatty acid sources.

In yet another embodiment, artificial sweeteners may be absent from the liquid human milk supplement. In yet another embodiment, artificial flavors may be absent from the liquid human milk supplement. In another embodiment, artificial food colours may be absent from the liquid human milk supplement.

The liquid human milk supplement may further comprise at least one selected from of a lipid component, a carbohydrate component, vitamins, minerals, and trace elements.

In another embodiment, the liquid human milk supplement may comprise medium-chain triglycerides (MCT's). In another embodiment, the liquid human milk supplement may further comprise unsaturated fatty acids, preferably essential unsaturated fatty acids.

In another embodiment, the lipid component of the liquid human milk supplement may comprise at least one selected from the group of medium-chain triglyceride, unsaturated omega-3 fatty acid, and omega-6 fatty acid. In another embodiment, the lipid component comprises at least one selected from the group of medium-chain triglycerides, arachidonic acid, docosahexaenoic acid, alpha-linolenic acid and linoleic acid. In another embodiment, the lipid component comprises at least two selected from the group of medium-chain triglycerides, arachidonic acid, docosahexaenoic acid, alpha-linolenic acid and linoleic acid. In another embodiment, the lipid component comprises at least three selected from the group of medium-chain triglycerides, arachidonic acid, docosahexaenoic acid, alpha-linolenic acid and linoleic acid. In another embodiment, the lipid component comprises medium-chain triglycerides, arachidonic acid, docosahexaenoic acid, alpha-linolenic acid and linoleic acid.

Preferably, the lipid component may comprise MCT's to an amount smaller than 70% of total lipid components. In another embodiment, the lipid component may comprise MCT's to an amount smaller than 50% of total lipid components. In yet another embodiment, the lipid component may comprise MCT's to an amount smaller than 40% of total lipid components. In another embodiment, the lipid component may comprise MCT's to an amount smaller than 30% of total lipid components. In another embodiment, the lipid component may comprise MCT's to an amount smaller than 20% of total lipid components. In another embodiment, the lipid component may comprise MCT's to an amount smaller than 10% of total lipid components.

Preferably the ratio of arachidonic acid to docosahexaenoic acid is in a range of 1.4 to 2.2, more preferably 1.6 to 2.0, most preferably the ratio is about 1.8. Preferably the ratio of linoleic acid to alpha-linolenic acid is in a range of 1.9 and 2.7, more preferably 2.1 and 2.5, most preferably the ratio is about 2.3.

In another embodiment, the carbohydrate component may comprise at least one selected from maltodextrin and lactose. In another preferred embodiment, the carbohydrate component does not comprise lactose.

In another embodiment, the liquid human milk supplement may further comprise at least one vitamin selected from the group of vitamin A, vitamin D, vitamin E, vitamin K1, vitamin C, vitamin B1, vitamin B2, niacin, vitamin B6, folate, pantothenic acid, vitamin B12, biotin. In another embodiment, the liquid human milk supplement may comprise vitamin A, vitamin D, vitamin E, vitamin K1, vitamin C, vitamin B1, vitamin B2, niacin, vitamin B6, folate, pantothenic acid, vitamin B12, and biotin.

In another embodiment, the liquid human milk supplement may comprise vitamin A in a range of 600 to 1800 IU/30ml supplement, vitamin D in a range of 100 to 800 IU/30ml supplement, vitamin E in a range of 3 to 9 IU/30ml supplement, vitamin K1 in a range of 3 to 15 µg/30ml supplement, vitamin C in a range of 10 to 40 mg/30ml supplement, vitamin B1 in a range of 70 to 250 µg/30ml supplement, vitamin B2 in a range of 100 to 325 µg/30ml supplement, niacin in a range of 700 to 2400 µg/30ml supplement, vitamin B6 in a range of 60 to 210 µg/30ml supplement, folate in a range of 17 to 70 µg/30ml supplement, pantothenic acid in a range of 0.3 to 1.2 mg/30ml supplement, vitamin B12 in a range of 0.1 to 0.5 µg/30ml supplement, and/or biotin in a range of 1.7 to 5.5 µg/30ml supplement. In another embodiment, the liquid human milk supplement may further comprise at least one mineral/trace element selected from the group of Na, K, Cl, Ca, P, Mg, Mn, Fe, I, Cu, Zn, Se, Cr, Mo, F. In another embodiment, the liquid human milk supplement may comprise Na, K, Cl, Ca, P, Mg, Mn, Fe, I, Cu, Zn, Se, Cr, Mo, and F.

In another embodiment, the liquid human milk supplement may comprise Na in a range of 10 to 60 mg/30ml supplement, K in a range of 10 to 70 mg/30ml supplement, Cl in a range of 10 to 60 mg/30ml supplement, Ca in a range of 30 to 150 mg/30ml supplement, P in a range of 13 to 85 mg/30ml supplement, Mg in a range of 1.5 to 8 mg/30ml supplement, Mn in a range of 2 to 16 µg/30ml supplement, Fe in a range of 0.03 to 3 mg/30ml supplement, I in a range of 7 to 40 µg/30ml supplement, Cu in a range of 15 to 80 µg/30ml supplement, Zn in a range of 0.3 to 2 mg/30ml supplement, Se in a range of 1.0 to 6 µg/30ml supplement, Cr in a range of 0.2 to 2 mg/30ml supplement, Mo in a range of 0.2 to 1.3 µg/30ml supplement, and/or F in a range of 0.2 to 4 µg/30ml supplement.

In another embodiment, the weight ratio of Ca to P may be in a range of 1.5 to 2.0, preferably in a weight ratio of about 1.75.

In another embodiment, the liquid human milk supplement may comprise Fe in a range of 0.06 to 2 mg/30ml supplement, preferably in a range of 0.1 to 0.8 mg/30ml supplement.

In another embodiment, Fe may be absent from the liquid human milk supplement.

In another embodiment, the liquid human milk supplement may further comprise at least one selected from the group of taurine, choline, carnitine, inositol. Preferably, the liquid human milk supplement may comprise taurine, choline, carnitine, and inositol. In another embodiment, the liquid human milk supplement may further comprise taurine in a range of 0.5 to 4 mg/30ml supplement, choline in a range of 5 to 30 mg/30ml supplement, carnitine in a range of 0.5 to 5 mg/30ml supplement, and/or inositol in a range of 1.5 to 7.3 mg/30ml supplement.

In another embodiment, the liquid human milk supplement further comprises at least one selected from the group of nucleotides, preservatives, antioxidants, pharmaceuticals, buffers, carotenoids, human milk oligosaccharides, probiotics, sialic-acid comprising materials.

In another embodiment, at least one selected from the group of nucleotides, preservatives, antioxidants, pharmaceuticals, buffers, carotenoids, human milk oligosaccharides, probiotics, sialic-acid comprising materials may be absent from the liquid human milk supplement.

Preferably, the liquid human milk supplement further comprises at least one selected from B vitamins, choline, cysteine, and methionine.

In other embodiments, the liquid human milk supplement further comprises vegetable oil, medium chain triglyceride, docosahexaenoic acid, and/or arachidonic acid.

The liquid human milk supplement may also comprise hydrolyzed or intact, naturally, and/or modified starch.

The present invention also relates to a single-dose dispenser comprising the liquid human milk supplement,
wherein the single-dose dispenser protects the liquid human milk supplement in an air-sealed and sterile environment,
wherein the single-dose dispenser comprises a compressible balloon that is connected to a tip,
wherein the tip comprises a closure system,
wherein the compressible balloon comprises a volume in a range of 0.5 to 5 ml, preferably 1 to 4 ml, more preferably 1 to 3 ml.

Preferably the single-dose dispenser comprises a twist-off closure. In another embodiment, the single dose dispenser may be composed of a polypropylene/ethylene-vinyl alcohol/polypropylene multilayer. In another embodiment, the single dose dispenser may be composed of a single polypropylene layer.

In another embodiment, the single-dose dispenser may be composed of a single or multi-layer material, wherein the single or multi-layer material has an oxygen transmission rate of less than 0.02 ml O₂/year. In another embodiment, the single-dose dispenser may be composed of a single or multi-layer material, wherein the single or multi-layer material has a light transmission of less than 0.5 % for wavelengths in a range of 420 to 700 nm. In another embodiment, the single-dose dispenser maybe packed into a secondary packing material, wherein the secondary packing material has a light transmission of less than 0.5 % for wavelengths in a range of 420 to 700 nm.

The present invention also relates to a multi-dose dispenser comprising the liquid human milk supplement,
wherein the multi-dose dispenser protects the liquid human milk supplement in an air-sealed and sterile environment,
wherein the multi-dose dispenser comprises a volume in a range 7.5 to 50 ml, preferably 10 to 30 ml, and
wherein the multi-dose dispenser dispenses adjustable volumes of 0.05 to 30 ml, preferably 0.5 to 30 ml of the liquid human milk supplement.

The present invention also relates to a use of the liquid human milk supplement for feeding infants, in particular low-birth-weight infants.

Preferably, the liquid human milk supplement is added to breast milk or breast milk substitute in a volume-to-volume ratio of 1-5 ml supplement to 15 ml milk.

Breast milk can be breast milk from the own mother, from wet-nurses, from milk banks, or human milk from commercial sources.

Breast milk substitute can be infant formula, hydrolyzed infant formula, or amino-acid based formula.

In another embodiment, the liquid human milk supplement is added to breast milk or breast milk substitute in a ratio of supplement to milk of about 1: 60. In another embodiment, the liquid human milk supplement is added to breast milk or breast milk substitute in a ratio of supplement to milk of about 1: 45. In another embodiment, the liquid human milk supplement is added to breast milk or breast milk substitute in a ratio of supplement to milk of about 1: 30. In another embodiment, the liquid human milk supplement is added to breast milk or breast milk substitute in a ratio of supplement to milk of about 1:10. In another embodiment, the liquid human milk supplement is added to breast milk or breast milk substitute in a ratio of supplement to milk of about 1:7.5. In another embodiment, the liquid human milk supplement is added to breast milk or breast milk substitute in a ratio of supplement to milk of about 1:5. In another embodiment, the liquid human milk supplement is added to breast milk or breast milk substitute in a ratio of supplement to milk of about 1: 3.5.

Preferably, the liquid human milk supplement is administered or delivered via a single-dose dispenser. It has been found that the single-dose dispenser is particularly useful for feeding low-birth-weight infants with weights higher 1500-1800g.

In another embodiment, the liquid human milk supplement is administered or delivered via a multi-dose dispenser. It has been further found that the multi-dose dispenser is particularly useful for feeding low-birth-weight infants with weights less than 1500-1800g and for feeding sick infants who are unable to suck.

Both single-dose and multi-dose dispensers allow to add individualized amounts of the liquid human milk supplement to human milk which is offered via syringes or other open/closed feeding devices suitable for low-birth-weight infants. In addition, individualized amounts can also be given directly into the mouth of the low-birth-weight infant once it has started suckling from the breast. Therefore, employing the dispenser systems for delivery of the fortifier to the LBW infant is a procedure that does not interfere with breastfeeding. The liquid human milk supplement may be applied, e.g. to LBW infants between 1500/1800 g to 3000g, immediately before, during, and after breastfeeding.

The liquid human milk supplement may also be given to malnourished infants as part of their nutrition rehabilitation therapy by using the single-dose and/or multi-dose dispenser.

The liquid human milk supplement may be added to or administered with a certain volume of infant milk, for example with about 150 ml breast milk or breast milk substitute. Indicated application doses may be added to the breast milk or breast milk substitutes. The volume of the infant milk, e.g. 150 ml, is a reference volume that is based on the fluid requirements per kg body weight of the LBW infant [3, 4, 5].

### 4. Brief description of the drawings

Preferred embodiments of the present invention are further described in the following detailed description, with reference to the following figures:
- Fig. 1: shows a 3D printer mockup of an example single-dose dispenser;
- Fig. 2: shows a technical drawing of an example single-dose dispenser;
- Fig. 3: shows a technical drawing of the pipette-like tip and of the compressible balloon of an example single-dose dispenser;
- Fig. 4: shows an example and a technical drawing of a multiple-dose dispenser;
- Fig. 5: shows application examples of multiple-dose dispensers.

### 5. Detailed description of the invention

Further preferred embodiments and examples of the invention are described hereinafter in detail with reference to the figures.

It is known that the fetus swallows amniotic fluid, which contains amino acids. Therefore, exposure to amino acids of the gut already starts before birth and contributes to maturation of the digestive system [7].

The present invention relates to liquid human milk supplements (fortifiers; hereinafter also referred to as the present invention), preferably having about 0.5 grams to 5.15 grams of amino acids. The supplements have relatively high concentrations of amino acids and are therefore concentrated.

The liquid human milk supplement comprises amino acids in free acid form. In addition to the essential-, semi-essential-, and non-essential amino acids, the supplements may include lipids, carbohydrates, vitamins, minerals, and trace elements, by selecting any one of the following variations: a) blends of essential, semi-essential, and non-essential amino acids, b) concentrates which are aseptically- or retort filled or packaged and c) in multi-dose dispensers which can deliver variable amounts of the supplement to feeding syringes/devices (hospital supply - enteral feeding period) or in single-dose dispensers which are used during the breastfeeding procedure (in home supply). The human milk supplements are especially suited for low-birth-weight (LBW) infants who are appropriate- (AGA) or small for their gestational age [SGA; 1], but may also be used in malnourished young infants. They are specifically useful in regional/local neonatal intensive care nurseries (NICUs), and other special care nurseries and they minimize the risk of microbiological contamination during preparation in institutional settings and when used at home.

The supplements of the present invention can comprise all essential-, semi-essential-, and non-essential amino acids for LBW infants who receive human milk.

Preferred examples of concentrations of amino acids (preferred; minimum and maximum) in the present invention for LBW infants with weights between 0.5-1.5 kg are presented in Table 1 of Example 1. Human milk fortified with the supplement provides at least twofold the amounts of all essential amino acids to infants between 0.5 - 1.5 kg that are necessary for growth and development. All amino acids used in the present invention are L-amino acids, preferably synthetically produced. Alternatively, the L-amino acids may be produced via fermentation. LBW infants receiving amino acids based supplements together with human milk are not at risk of sensitization to cow's milk, other proteins, and exposure to peptide chains with sizes > 1000 Dalton (from hydrolyzed protein). The supplement is hypoallergenic.

The supplement of the invention is prepared by mixing the amino acids and optionally other components with water. The water is preferably doubly distilled water. In another embodiment, a specific pH value of the liquid human milk supplement may be adjusted. In a preferred embodiment, the pH is adjusted to a pH value within the range of pH 4 to pH 7.

Human milk fortifiers are commercially available as powders to be reconstituted in pumped- or donor human milk or in concentrated liquid form that can be added to human milk prior to feeding. The use of those fortifiers requires that the mother has to pump her milk from the breast prior to mixing it with the fortifier, which interferes with the natural way of breastfeeding. Examples of such fortifiers are mentioned: EP1871182B1/US20060204632, EP2152089B1/US2008286414A and WO2010/134810A1. Once LBW infants have reached a weight of approximately 1500-1800 grams and a certain developmental maturity, they start nutritive suckling and subsequently can be breastfed like term infants. The present invention is thus directed to a human milk supplement (fortifier) in the form of a concentrated liquid, which preferably can be applied by a) employing single-dose dispensers during each breastfeeding procedure (in-home supply), and b) employing a multi-dose dispenser according to the needs of the LBW infant during the enteral nutrition period (hospital supply).

The preferred device mentioned under a) for delivery of the supplement during the breastfeeding procedure (body weight > 1500 (1800) g) is a soft plastic single-serving dispenser with a compressible balloon which is connected to a pipette like tip.

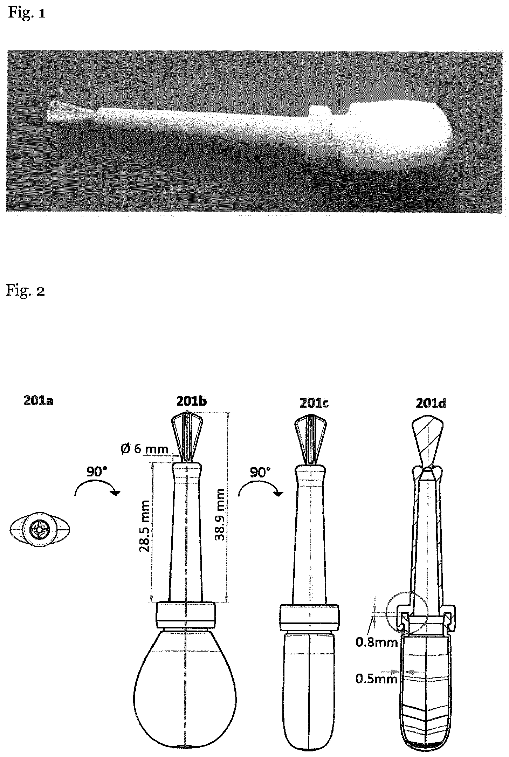

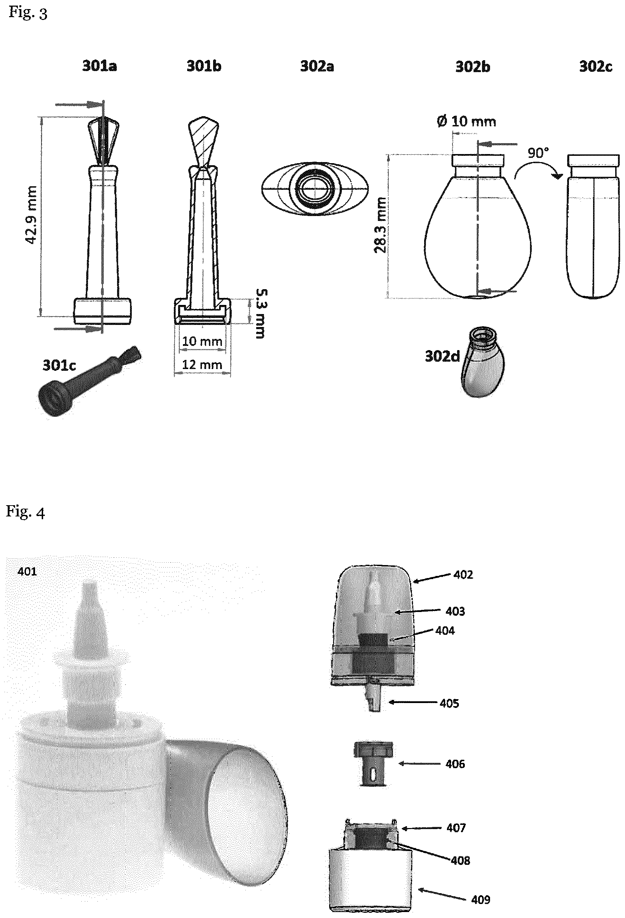

Fig.1 illustrates a 3D printer mockup of a single-dose dispenser. The single-dose dispenser may be made, for example, from a PP/EVOH/PP multilayer or a single layer of polypropylene material. It comprises a pipette-like tip with a twist-off closure connected to a balloon reservoir. Fig. 2 illustrates a technical drawing of the single-dose dispenser with a top-view (indicated with the reference numeral 201a), a side-view (201b), a further side-view after 90°C rotation (201c), and an inside view (201d) of the dispenser. Exemplary, not-limiting dimensions are indicated. Fig.3 contains further details on the pipette-like tip (301a, 301b, 301c) and the compressible balloon (302a, 302b, 302c, 302d). 301a illustrates the side view of the pipette like tip with a twist-off closure, 301b illustrates the inside view of the pipette like tip. 301c illustrates a 3D model of the pipette like tip. 302a illustrates the top-view of the compressible balloon that is connected to the pipette like tip. The compressible balloon with dimensions as indicated in 302b comprises a volume of 2.2 ml. In general, the dimensions of the compressible balloon may be chosen such that other volumes, e.g. in a range of 0.5 ml to 5 ml, of the liquid human milk supplement may be squeezed out. 302C illustrates the side view of the balloon, indicating the asymmetry of the curvatures of the balloon. 302d presents a 3D view of the compressible balloon.

By using the single-dose dispenser, as illustrated in Figures 1, 2, and 3, a certain amount of the supplement may be given directly into the mouth of the suckling infant via compressing the balloon and directing the pipette-like tip to the mouth of the infant (in-home supply).

By squeezing out the balloon the supplement is directly applied into the mouth of the LBW infant during breastfeeding. The preferred device mentioned under a) is a further development of the "finger feeder" dispenser system known in the art. The device of the present invention which is mentioned under a) is a new and safe in-home method of providing supplements to infants, especially LBW infants, during, before, and after breastfeeding. Therefore, the way how the supplement is provided by employing the preferred device mentioned under a) does not interfere with the natural, recommended, and preferred way to feed infants.

The preferred device mentioned under b) for delivery of the supplement, e.g. during the enteral (tube) feeding period (weight range of LBW infants 500g - 1500/1800g) preferably is a multi-dosage dispenser (7.5 - 50ml) similar to a patented device (EP1674399B1) where specified volumes or exact numbers of drops can be added to human milk which is generally provided by enteral feeding syringes or other open/closed systems. The supplement can be added to human milk according to feeding tolerance and individual needs of the LBW infant.

Fig.4 illustrates an exemplary multi-dose dispenser (401). The multi-dose dispenser comprises a cover (402), a push-button (403), a snap on with anti-activation feature (404), a plunger (405), a LDPE snapping part (406), an upper cap with thread (407), a delaminating bag-in-bottle (408), and a base cap (409).

By employing a multi-dose dispenser, variable amounts of the supplement can be added to human milk which is provided by enteral feeding syringes or other open/closed feeding systems (hospital supply).

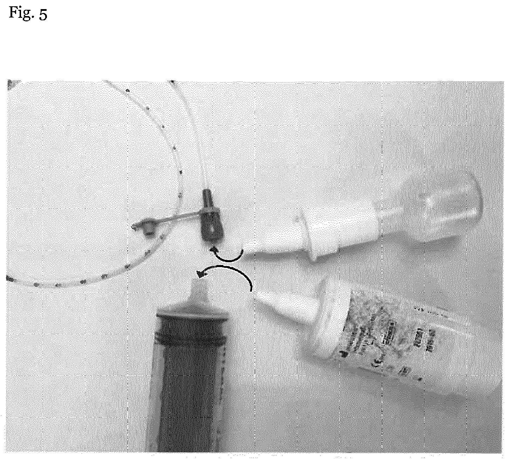

Fig.5 illustrates possible connections of the multi-dose dispenser for hospital supply. Multi-dose dispensers may be connected to syringes or feeding tubes that allow enteral feeding of LBW infants with weights below e.g. 1500/1800 grams.

The present invention also includes aseptically packaged, which are preferred, or retort-packaged embodiments having improved stability. The aseptically packaged embodiments of the present invention can be prepared or manufactured using any of a variety of techniques well known to those of ordinary skill in the formulation art (e.g. using filling lines). Suitable aseptic packaging techniques include any of the well-known aseptic filling/packaging methods disclosed in the formulation arts for preparing liquid nutritional formulas, all of which are generally directed to the sealing or filling of a sterilized liquid into a sterile container. The aseptically packaged embodiments of the present invention may include any container or package suitable for use with liquid nutritional products and also capable of withstanding aseptic processing conditions (e.g., sterilization). Preferred examples of containers are multi- and single-dose dispensers and other non-limiting examples of such containers include single or multi- or single-use bags, plastic bottles or containers, pouches, metal cans glass bottles, foil or other flexible pouches, syringes, vials, or any other container meeting the above-described criteria. The aseptically packaged container for these embodiments is typically sterilized prior to being filled with its sterilized contents. The present invention is also directed to a method of reducing the risk of microbial contamination of infant feeding compositions. Microbiological quality of the supplements (fluid or gel) needs to be maintained during the whole shelf-life.

The supplements of the present invention can further comprise or consist of the essential elements and limitations of the invention described herein, as well as any additional or optional ingredients, components, or limitations useful in infant nutrition applications.

The supplement, in addition to the amino acids, can comprise carbohydrates, and lipids as macronutrients of sufficient types and amounts that when used in combination with human milk or other infant feeding formula, help to meet the nutritional needs of the infant, especially the LBW infant. The macronutrients in the various embodiments of the present invention are described in Table 2 of Example 2. The energy density of breast-milk requires that LBW infants be fed with volumes of milk that are too high to be tolerated by those infants. LBW infants require 100 - 130 kcal/kg/day [3, 4]. In order to provide a caloric intake, which meets the needs of LBW infants in a volume they can tolerate, the caloric content of breast-milk can be supplemented with amino acids, fat and carbohydrates.

The supplement can comprise a carbohydrate suitable for use in infants, especially LBW infants, in particular hydrolyzed or intact, naturally and/or modified, starches. Other non-limiting examples of suitable carbohydrate sources include hydrolyzed cornstarch, maltodextrin (i.e. non-sweet, nutritive polysaccharide having a DE value less than 20), glucose polymers, sucrose, corn syrup, corn syrup solids (i.e., polysaccharide having a DE value greater than 20), glucose, rice syrup, fructose, high fructose corn syrup, and indigestible human milk oligosaccharides (HMOs). The carbohydrates may comprise lactose or can be substantially free of lactose.

The supplement also can comprise a lipid component suitable for use in infants, especially LBW. Lipids suitable for use in the present invention may include coconut oil, soy oil, corn oil, olive oil, safflower oil, high oleic safflower oil, MCT oil (medium chain triglycerides), sunflower oil, high oleic sunflower oil, structured triglycerides, palm and palm kernel oils, palm olein, canola oil, marine oils, cottonseed oils, and combinations thereof. Suitable fats for use in the liquid supplement include emulsifiers to help the various fortifier components readily disperse when combined with human milk. Non-limiting examples of suitable emulsifiers include soya bean lecithin, polyoxythylene stearate, polyoxyethylene sorbitan mono-oleate, polyoxyethylene sorbitan monopalmitate, polyoxyethylene sorbitan monostearate, ammonium phosphatides, polyoxyethylene sorbitan monolaurate, citric acid esters of mono and diglycerides of fatty acids, tartaric acid esters of mono and diglycerides of fatty acids, and combinations thereof. Natural soy lecithin is especially useful in this respect. The lipid component may include emulsifiers suitable for use in infant nutritional products.

The present invention also includes those embodiments that comprise as part of the fat component one or more of arachidonic acid, docosahexaenoic acid, or combinations thereof, alone or in further combination with linoleic acid, linolenic acid, or both. The weight ratio of arachidonic acid: docosahexaenoic acid preferably should be between 1.0 - 2.0 [3]. The supplement will be with oils that can be made using standard techniques known in the art.

The supplement can comprise vitamins, minerals, and trace minerals as recommended by regulatory bodies and international nutrition committees [3, 4]. It may comprise any of a variety of vitamins, non-limiting examples of which include vitamin A, vitamin D, vitamin E, vitamin K, thiamine (vitamin B1), riboflavin (vitamin B2), pyridoxine (vitamin B2), vitamin B12, niacin, folic acid, pantothenic acid, biotin, vitamin C, choline, inositol, salts and derivatives thereof, and combinations thereof. An example how much minerals, vitamins, and trace elements can be added with the liquid human milk supplement to 150 ml of human milk is provided in Table 2.

The supplement can be with levels of vitamins B2, B6, B12, folate, as well as methionine, cysteine, and choline, which are crucial co-factors and substrates for many basic biological pathways including one-carbon metabolism, and in LBW infants they are particularly important for brain function and development and epigenetic mechanisms. These are essential nutrients that cannot be synthesized endogenously and thus need to be taken by LBW infants via the diet [8]. In particular, this can be important for LBW infants in developing countries, where mothers more often suffer from folate- and vitamin B12 deficiencies [9].

In LBW infants of vitamin D-deficient mothers, a vitamin D intake of 800 to 1500 IU/day is necessary to reach a circulating 25(OH) vitamin D concentration above 75 nmol/L, which rules out osteopenia and rickets. During metabolic balance studies, calcium net absorption increased from 50 % to 71 % by feeding appropriate-for-gestational age preterm infants weighing <1500 g banked human milk alone or supplemented with 30 mg/day (1200 IU) without calcium fortification [3]. Nutrition committees therefore recommend vitamin D intakes between 800-1000 IU/day [3, 4]. Because vitamin D concentration in breast milk is low, the present invention provides 100 - 800; preferable 300 IU vitamin D3 to be added to 150ml of human milk to satisfy the requirements of LBW infants.

The supplement may also further comprise any of a variety of minerals known or otherwise suitable for us in infant or other nutritional formulas, non-limiting examples of which include phosphorus, magnesium, calcium, zinc, manganese, copper, iodine, sodium, potassium, chloride, selenium, iron and combinations thereof. The calcium : phosphorus ratio (mg : mg) in the supplement may be between 1.5 and 2.0, preferably 1.75. An example how much minerals, trace elements, choline, inositol, and taurine can be added to 150 ml of human milk is provided in Table 2.

In one embodiment (weight range 500g - 1500 (1800) g), the amount of iron which is added to 150 ml of breast milk can be as low as between as 0.1-2 mg. This is because VLBW are extremely prone to infections and iron should not be given in such situations. Moreover, VLBW frequently receive blood transfusions and can be overloaded with iron. Iron supplements for the rapidly growing LBW at the latest are necessary if their birthweight has doubled. Therefore, in another embodiment (weight > 1500 (1800) g) the amount of iron which is added to 150ml of human milk can be 2.0-4.5mg.

The supplement may further comprise other optional ingredients that may modify the physical, chemical, aesthetic or processing characteristics of the formulas or serve as pharmaceutical or additional nutritional components when used in the targeted population. Many such optional ingredients are known for use in food and nutritional products, including infant formulas, and may also be used in the present invention, provided that such optional materials are compatible with the essential materials described herein, are safe and effective for their intended use, do not otherwise unduly impair product performance, and are accepted by the regulatory bodies. Non-limiting examples of such optional ingredients include preservatives, antioxidants, various pharmaceuticals, buffers, carotenoids, colorants, flavors, nucleotides and nucleosides, thickening agents, stabilizers, human milk oligosaccharides, probiotics, sialic acid-containing materials, and other excipients or processing aids.

The invention is further explained by the following examples without limiting it.

### Example 1:

A liquid human milk supplement according to the invention is prepared by adding free amino acids in amounts as described in Table 1 to double-distilled water. The amino acids are given in the three-letter code. The supplements are suitable for LBW infants (ca. 0.5-1.5 (1.8) kg) and may be added to 150 ml of breast milk or breast milk substitute. The volume of the supplement is 30 ml.

**Table 1**

| Liquid human supplement comprising amino acids | | | |
|---|---|---|---|
| Amino acid | | Amount of amino acid in supplement | |
| | Minimum (ca.25 %) | Optimum (100%) | Maximum (ca.250%) |
| **Nutrient in (mg)** | | | |
| **total amino acids** | **513** | **2053** | **5132** |
| **essential (mg)** | | | |
| LEU | 50 | **201** | 503 |
| ILE | 25 | **108** | 270 |
| VAL | 35 | **152** | 380 |
| LYS | 50 | **205** | 515 |
| MET | 19 | **78** | 200 |
| THR | 25 | **105** | 265 |
| PHE | 29 | **117** | 295 |
| HIS | 18 | **75** | 190 |
| TRP | 13 | **52** | 130 |
| ∑ (essential) | 264 | **1093** | 2748 |
| **semi-essential** | | | |
| TYR | 11 | **46** | 115 |
| CYS | 8 | **35** | 88 |
| GLY | 50 | **208** | 520 |
| GLU/AMATE | 50 | **211** | 528 |
| ARG | 35 | **145** | 365 |
| ∑ (semi-essential) | 154 | **645** | 1616 |
| non-essential | | | |
| ALA | 25 | **102** | 255 |
| ASP | 15 | **65** | 163 |
| PRO | 29 | **117** | 293 |
| SER | 7 | **31** | 78 |
| ∑ (non-essential) | 76 | **315** | 789 |

### Example 2:

The liquid human milk supplement of Example 1 is prepared, wherein the additional lipid, carbohydrate, vitamin and other supplements according to Table 2 are added. The supplements with macro/micronutrients are suitable for LBW infants (ca. 0.5-2.5 kg) and may be added to 150 ml of breast milk or breast milk substitute. The volume of the supplement is 30 ml.

**Table 2**

| Supplements with additional components to the amino acid composition of table 1 | |
|---|---|
| **Total calories (kcal)** | **25.6** |
| **Amino acids of Table 1 (g)** | **2.053** |
| **Lipids (g)** | **1.08** |
| Medium-chain triglycerides (g) | 0.7 |
| Percentage of medium-chain triglycerides in total lipid amount (%) | 65 |
| Arachidonic acid (mg) | 16.8 |
| Docosahexaenoic acid (mg) | 9.4 |
| Ratio of arachidonic acid : docosahexaenoic acid | 1.8 |
| Linoleic acid (mg) | 57.5 |
| alpha-Linolenic acid (mg) | 25 |
| Ratio of linoleic acid : alpha-linolenic acid | 2.3 |
| **Carbohydrates (g)** | **1.95** |
| Lactose (g) | 0 |
| Maltodextrin (g) | 1.95 |
| **Minerals** | |
| Na (mg) | 55.08 |
| K (mg) | 60 |
| Cl (mg) | 48.18 |
| Ca (mg) | 113.4 |
| P (mg) | 64.95 |
| Ca/P ratio | 1.74 |
| Mg (mg) | 6 |
| Mn (ug) | 12.12 |
| Fe (mg) | 2.7 |
| I (ug) | 25.38 |
| Cu (ug) | 75 |
| Zn (mg) | 1.41 |
| Se (ug) | 5.58 |
| Cr (mg) | 1.38 |
| Mo (ug) | 1.2 |
| F (ug) | 3.6 |
| **Vitamins** | |
| A (IU) | 1774.5 |
| D (IU) | 300 |
| E (IU) | 8.955 |
| K1 (ug) | 12 |
| C (mg) | 30 |
| B1 (ug) | 225 |
| B2 (ug) | 300 |
| Niacin (ug) | 2250 |
| B6 (ug) | 195 |
| Folate (ug) | 60 |
| Pantothenic acid (mg) | 1.05 |
| B12 (ug) | 0.4 |
| Biotin (ug) | 5.25 |
| **Further ingredients** | |
| Choline (mg) | 25 |
| Inositol (mg) | 6.66 |
| Taurine (mg) | 3 |
| Carnitine (mg) | 3.96 |

### References

1) MS Kramer - Bulletin of the World Health Organization, 1987 - ncbi.nlm.nih.gov
2) https://intergrowth21.tghn.org/.../intergrowth-21st-fetal-growth-s
3) Agostoni C et al for the ESPGHAN COMMITTEE on Nutrition. Enteral Nutrient Supply for Preterm Infants: Commentary From the European Society of Paediatric Gastroenterology, Hepatology and Nutrition Committee on Nutrition. Journal of Pediatric Gastroenterology & Nutrition:January 2010; 50: 85-91 doi: 10.1097/MPG.obo13e3181adaeeo
4) Sourabh Dutta,* Balpreet Singh, Lorraine Chessell, et al Guidelines for Feeding Very Low Birth Weight Infants Nutrients. 2015 Jan; 7(1): 423-442. . doi: 10-3390/nu7010423
5) Ziegler EE. Preterm and low-birth-weight infants. World Rev Nutr Diet. 2015;113:214-7. doi: 10.1159/000360342. Epub 2015 Apr 13. Review.
6) Haschke F, Grathwohl D, Haiden N. Metabolic Programming: Effects of Early Nutrition on Growth, Metabolism and Body Composition. iIn: Bhatia J, Shamir R, Vandenplas Y (eds): Protein in Neonatal and Infant Nutrition: Recent Updates. Nestle Nutr Inst Workshop Ser, vol 86, pp 87-95, (DOI: 10.1159/000442728)
7) Underwood MA1, Gilbert WM, Sherman MP. Amniotic fluid: not just fetal urine anymore. Journal of Perinatology (2005) 25, 341-348. doi:10.1038/sj.jp.7211290
8) Oosterink JE, Naninck EF, Korosi A et al. Accurate measurement of the essential micronutrients methionine, homocysteine, vitamins B6, B12, B9 and their metabolites in plasma, brain and maternal milk of mice using LC/MS ion trap analysis. J Chromatogr B Analyt Technol Biomed Life Sci. 2015 Aug 15;998-999:106-13. doi: 10.1016/j.jchromb.2015.07-008.
9) Yajnik CS, Deshmukh US Fetal programming: maternal nutrition and role of one-carbon metabolism. Rev Endocr Metab Disord. 2012 Jun;13(2):121-7. doi: 10.1007/s11154-012-9214-8

## Claims

1. A liquid human milk supplement comprising at least the amino acids leucine, isoleucine, valine, lysine, methionine, threonine, phenylalanine, histidine, and tryptophan,
wherein the total amount of amino acids comprises at least 25 % of the caloric content of the liquid human milk supplement.

2. The liquid human milk supplement of claim 1, wherein the total amount of amino acids comprises at least 40 % of the caloric content of the liquid human milk supplement, in particular at least 50 % of the caloric content of the liquid human milk supplement.

3. The liquid human milk supplement of claim 1 or 2, further comprising at least one semi-essential amino acid, in particular at least one semi-essential amino acid selected from tyrosine, cysteine, glycine, glutamic acid and arginine, preferably all said semi-essential amino acids.

4. The liquid human milk supplement of any of claims 1 to 3, further comprising at least one non-essential amino acid, in particular at least one non-essential amino acid selected from alanine, aspartic acid, proline and serine, preferably all said non-essential amino acids.

5. The liquid human milk supplement of any of claims 1 to 4, wherein the amino acids are present in the following amounts per 30 ml liquid human milk supplement:
leucine in a range of 50 to 503 mg, preferably 100 to 400 mg, more preferably 150 to 300 mg,
isoleucine in a range of 25 to 270 mg, preferably 55 to 210 mg, more preferably 80 to 160 mg,
valine in a range of 35 to 380 mg, preferably 75 to 305 mg, more preferably 115 to 230 mg,
lysine in a range of 50 to 515 mg, preferably 100 to 410mg, more preferably 150 to 307 mg,
threonine in a range of 25 to 265 mg, preferably 52 to 210 mg, more preferably 78 to 157 mg,
phenylalanine in a range of 29 to 295 mg, preferably 58 to 235 mg, more preferably 87 to 175 mg,
methionine in a range of 19 to 200 mg, preferably 40 to 156 mg, more preferably 58 to 117 mg,
histidine in a range of 18 to 190 mg, preferably 37 to 150 mg, more preferably 56 to 112 mg, and
tryptophan in a range of 13 to 130 mg, preferably 26 to 104 mg, more preferably 39 to 78 mg.

6. The liquid human milk supplement of any of claims 1 to 5, wherein the amino acids, if present, are present in the following amounts per 30 ml liquid human milk supplement:
tyrosine in a range of 11 to 115 mg, preferably 23 to 92 mg, more preferably 34 to 69 mg,
cysteine in a range of 8 to 88 mg, preferably 17 to 70 mg, more preferably 26 to 52 mg,
glycine in a range of 50 to 520 mg, preferably 100 to 410 mg, more preferably 150 to 310 mg,
glutamic acid in a range of 50 to 528 mg, preferably 105 to 420 mg, more preferably 160 to 315 mg,
arginine in a range of 35 to 365 mg, preferably 72 to 290 mg, more preferably 110 to 215 mg,
alanine in a range of 25 to 255 mg, preferably 50 to 204 mg, more preferably 75 to 155 mg,
aspartic acid in a range of 15 to 163 mg, preferably 30 to 130 mg, more preferably 50 to 100 mg,
proline in a range of 29 to 293 mg preferably 58 to 235 mg, more preferably 87 to 175 mg, and
serine in a range of 7 to 78 mg, preferably 15 to 62, more preferably 23 to 46 mg.

7. The liquid human milk supplement of any of claims 1 to 6, further comprising at least one selected from of a lipid component, a carbohydrate component, vitamins, minerals, trace elements.

8. The liquid human milk supplement of any of claims 1 to 7, further comprising at least one selected from B vitamins, choline, cysteine, and methionine.

9. The liquid human milk supplement of any of claims 1 to 8, further comprising vegetable oil, medium chain triglycerides, docosahexaenoic acid, arachidonic acid, linoleic acid and/or alpha-linolenic acid.

10. A single-dose dispenser comprising the liquid human milk supplement according to any of claims 1 to 9,
wherein the single-dose dispenser protects the liquid human milk supplement in an air-sealed and sterile environment,
wherein the single-dose dispenser comprises a compressible balloon that is connected to a tip,
wherein the tip comprises a closure system,
wherein the compressible balloon comprises a volume in a range of 0.5 to 5 ml, preferably 1 to 4 ml, more preferably 1 to 3 ml.

11. A multi-dose dispenser comprising the liquid human milk supplement according to any of claims 1 to 9,
wherein the multi-dose dispenser protects the liquid human milk supplement in an air-sealed and sterile environment,
wherein the multi-dose dispenser comprises a volume in a range 7.5 to 50 ml, preferably 10 to 30 ml, and
wherein the multi-dose dispenser is adapted to dispense adjustable volumes of 0.5 to 50 ml, preferably 0.5 to 30 ml of the liquid human milk supplement.

12. Use of the liquid human milk supplement of any of claims 1 to 9 for feeding infants, in particular low-birth-weight infants.

13. Use of the liquid human milk supplement according to claim 12, wherein the liquid human milk supplement is added to breast milk or breast milk substitute in a volume-to-volume ratio of 1 ml-5 ml supplement to 15 ml milk,
in particular, wherein the breast milk is breast milk from the mother, from wet-nurses, from milk banks, or human milk from commercial sources, and wherein the breast milk substitute is infant formula, hydrolyzed infant formula, or amino-acid based formula.

14. Use of the liquid human milk supplement according to claim 12 or 13,
wherein the liquid human milk supplement is administered via a single-dose dispenser as defined in claim 10.

15. Use of the liquid human milk supplement according to claim 12 or 13,
wherein the liquid human milk supplement is administered via a multi-dose dispenser as defined in claim 11.
